(19) Europäisches Patentamt
European Patent Office
Office européen des brevets

(11) **EP 3 960 193 A1**

(12) **EUROPEAN PATENT APPLICATION**

(43) Date of publication:
**02.03.2022  Bulletin 2022/09**

(21) Application number: **20192425.5**

(22) Date of filing: **24.08.2020**

(51) International Patent Classification (IPC):
**A61K 35/748** (2015.01)        **A61P 9/00** (2006.01)
**A61P 31/04** (2006.01)        **A61L 27/36** (2006.01)

(52) Cooperative Patent Classification (CPC):
**A61K 35/748; A61L 27/3637; A61L 31/005;
A61P 9/00; A61P 31/04**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Brandenburgische Technische
Universität
Cottbus-Senftenberg
01968 Senftenberg (DE)**

(72) Inventors:
• **KÜPPER, Jan-Heiner
01968 Kleinkoschen (DE)**
• **Jung, Friedrich
01259 Dresden (DE)**

(74) Representative: **Simandi, Claus
Badehausallee 55
27476 Cuxhaven (DE)**

(54) **ARTHROSPIRA FOR USE IN THE TREATMENT OF DISEASES**

(57)    The present invention refers to beneficial effects of an extract of Arthrospira with respect to endothelial cells and related diseases.

Hereto, in a first aspect, the present invention relates to a coating comprising an extract of Arthrospira as an active ingredient for implantable devices due to said beneficial effects.

Hereto, in a second aspect, the present invention relates to a pharmaceutical preparation / composition comprising an extract of Arthrospira as an active ingredient for therapeutic and prophylactic treatment of bacterial infections, preferably bloodstream infections, primary bacteremia and sepsis due to said beneficial effects.

Figure 1:

**Description**

The present invention refers to beneficial effects of an extract of Arthrospira with respect to endothelial cells and related diseases.

Hereto, in a first aspect, the present invention relates to a coating comprising an extract of Arthrospira as an active ingredient for implantable devices due to said beneficial effects.

Hereto, in a second aspect, the present invention relates to a pharmaceutical preparation / composition comprising an extract of Arthrospira as an active ingredient for therapeutic and prophylactic treatment of bacterial infections, preferably bloodstream infections, primary bacteremia and sepsis due to said beneficial effects.

Arthrospira is a genus of free-floating filamentous multicellular, photosynthesizing cyanobacterium characterized by cylindrical, multicellular trichomes in an open left-hand helix. A dietary supplement is made from A. platensis and A. maxima, known as spirulina. Arthrospira, in particular spirulina is rich in proteins, vitamins, essential amino acids, minerals and essential fatty acids [1]. Beyond its use as forage with known effects on flesh, egg and plumage color, milk yield and fertility, it has been found to have additional pharmacological properties. Many preclinical and a few clinical studies suggest several therapeutic effects ranging from reduction of cholesterol to enhancement of the immune system, an increase in intestinal lactobacilli, a detoxification of heavy metals and drugs and anti-oxidant, anti-inflammatory properties [1-4].

The excessive generation of intracellular reactive oxygen species (ROS) has been implicated in the pathogenesis of many cardiovascular diseases, including atherosclerosis, hypertension, heart failure and the associated endothelial dysfunction [5]. Dartsch reported a dose-dependent inactivation of free superoxide radicals (anti-oxidant effect) as well as an anti-inflammatory effect characterized by a dose-dependent reduction of the metabolic activity of neutrophils and a dose-dependent inactivation of superoxide radicals generated during an oxidative burst [5]. In addition, Chu et al. could show that the aqueous extract of spirulina had a protective effect against apoptotic cell death due to free radicals in fibroblasts [6]. This antioxidant potential has been attributed mainly to phycocyanin prepared from spirulina by aqueous extraction [7, 8]. A purified peptide from Arthrospira, in particular spirulina inhibited the Angiotensin II induced production of intracellular reactive oxygen species (ROS) in a human endothelial cell line [9]. Moreover, the changes in cell morphology correlated with intracellular ROS production and were recovered by treatment with the purified peptide.

These findings were confirmed in an animal study in rats, in which the protective effect of spirulina against 4-nitroquinoline-1-oxide (4NQO) induced hepato- and nephron-toxicity was explored. The 4NQO administration increased the oxidative stress with a concomitant decline in the levels of non-enzymic [reduced glutathione (GSH)] and enzymic antioxidants [(Superoxide dismutase (SOD), Catalase (CAT), Glutathione peroxidase (GPx), and Glutathione-S-transferase (GST)] in both liver and kidney and resulted in increased levels of hepatic and renal markers [Alanine Transaminase (ALT), Aspartate Transaminase (AST), Lactate Dehydrogenase (LDH), urea, creatinine and uric acid] in the serum of experimental animals. The oral pretreatment with aqueous extract of spirulina prevented those 4NQO-induced changes in the levels of hepatic and kidney enzymes in the serum of experimental rats. It counteracted the 4NQO induced lipid peroxidation and maintained the hepatic and kidney antioxidant defense system at near normal in both liver and kidney [10].

However, the state of the art does not refer to the beneficial effects of an extract of Arthrospira, in particular spirulina with respect to endothelial cells.

The endothelial cell (EC) monolayer is a crucial component of the normal vascular wall, providing an interface between the bloodstream and the surrounding tissue of the blood vessel wall. ECs are also involved in physiological events including angiogenesis, inflammation and the prevention of thrombosis. It is evident that each phase of the vascular response to injury is influenced or may be controlled by the endothelium. Thus, disturbances of endothelial functions by toxic endogenous or exogenous substances such as produced by certain bacteria can have dramatic physiological consequences. Moreover, ECs have been encouraged to grow on the surface of stents by local delivery of vascular endothelial growth factor (VEGF), an endothelial cell mitogen, after implantation of a stent.

Therefore, the inventors have conducted a study in order to analyze whether an extract of Arthrospira, in particular spirulina may influence the endothelial cell monolayer formation in tissue culture plates and whether Arthrospira, in particular spirulina could affect the toxic influence of bacterial toxins like lipopolysaccharides (LPS) on primary human venous endothelial cells (HUVEC).

Surprisingly, the inventors have found that an extract of Arthrospira, in particular spirulina has a protective and curative effect with respect to endothelial cells and the endothelial cell monolayer formation. Such an advantageous effect of an extract of spirulina is well supported by the presented examples and figures.

Accordingly, the present invention is directed to the use of an extract of Arthrospira, in particular spirulina in the manufacture of a medicament or a dietary supplement for use in treating or preventing a disease or condition benefiting from said protective and curative effect with respect to endothelial cells and the endothelial cell monolayer formation.

Moreover, there is a large need to provide novel applications for an extract of Arthrospira, in particular spirulina in order to exploit its healthy and curative capacity.

An extract of spirulina is an advantageously safe and natural composition without any known side effects and disadvantages.

Hence, an extract of Arthrospira, in particular spirulina is useful for a clinical or therapeutically interaction with endothelial cells and related diseases, in particular using implantable devices for the prophylaxis and treatment of such diseases.

The term "Arthrospira" in accordance with the present invention shall mean a genus of free-floating filamentous multicellular, photosynthesizing cyanobacterium characterized by cylindrical, multicellular trichomes in an open left-hand helix. A dietary supplement is preferably made from biomass of A. platensis and/or A. maxima, known as spirulina. Spirulina is commonly used and a commercial name of a variety of similar cyanobacterial species belonging to the genus Arthrospira. The genus Arthrospira includes but is not limited to the following species: A. platensis, A. maxima, A. fusiformis, A. indica, A. innermongoliensis, A. jenneri, A. massartii and A. erdosensis. In accordance with the present invention the following species are preferred, namely A. platensis, A. maxima and A. fusiformis or commercially available spirulina.

An extract of Arthrospira, in particular spirulina may be preferably obtained by aqueous extraction [6], cf. example 2. Such aqueous extract can be commercially obtained from e.g. Sigma Aldrich, Munich, Germany.

In a further preferred embodiment of the invention the extract of Arthrospira, in particular spirulina is enriched with bioactive preferably own ingredients or compounds of the native biomass, e.g. by means of concentration, fractionation or addition. In particular, such bioactive compounds are selected from the group of secondary plant substances, preferably photosynthetically active pigments, phycocyanin, xanthophyll, chlorophyll, beta-carotene, echinenone, xanthine, fatty acids, linolenic acid (ALA), linoleic acid, stearidonic acid (SDA), eicosapentaenoic acid (EPA), docosahexaenoic acid (DHA), arachidonic acid (AA), oligosaccharides, and polyphenols.

Implantable devices include, for example, stents, stent-grafts, synthetic bypass grafts, embolic filters, occluder systems, detachable coils, pacemaker and defibrillator leads, plates, screws, spinal cages, dental implants, ventricular assist devices, artificial hearts, artificial heart valves, annuloplasty devices, artificial joints, and implantable sensors. Frequently, implanted medical apparatus must be designed to be sufficiently biocompatible to the host body.

Otherwise, the body will manifest a rejection of the implant by way of a thrombotic, inflammatory or other deleterious response. Moreover, restenosis involves recoil and shrinkage of the vessel. Subsequently, recoil and shrinkage of the vessel are followed by proliferation of medial smooth muscle cells in response to injury of the vessel. In response to blood vessel injury, smooth muscle cells in the tunica media and fibroblasts of the adventitial layer undergo phenotypic change which results in the secretion of metalloproteases into the surrounding matrix, luminal migration, proliferation and protein secretion. Various other inflammatory factors are also released into the injured area including thromboxane A2, platelet derived growth factor (PDGF) and fibroblast growth factor (FGF).

Such implantable devices, therefore, are designed or fabricated from materials possessing surface properties that minimize bodily response at the tissue device interface and an injury caused to the tissue may occur during the implantation procedure (e.g. percutaneous transluminal coronary balloon angioplasty (PTCA), etc.), in particular the endothelial cell monolayer may be injured in the blood vessels.

Endothelial cell growth factors and environmental conditions in situ are therefore essential in modulating endothelial cell adherence, growth and differentiation at the site of blood vessel injury. Accordingly, with respect to restenosis and other blood vessel diseases, there is a need for the development of new methods and compositions for coating medical devices, which would promote and accelerate the formation of a functional endothelium on the surface of implanted devices so that a confluent EC monolayer is formed on the target blood vessel segment or grafted lumen and preventing or treating neo-intimal hyperplasia, or preventing or treating restenosis or preventing or treating thrombosis or preventing or treating embolism.

The present invention provides a medical device for implanting into the lumen of a blood vessel or an organ with a lumen as disclosed in the claims. The medical device comprises a coating comprising an extract of Arthrospira, in particular spirulina.

Hence, the present invention refers to a pharmaceutical composition comprising an extract of Arthrospira, in particular spirulina for use in the prevention or treatment of a blood vessel disease selected from the group of neo-intimal hyperplasia, restenosis, thrombosis or embolism.

In particular, the present invention refers to a pharmaceutical composition comprising an extract of Arthrospira, in particular spirulina for use in the regeneration, in particular re-endothelization and / or acceleration of forming endothelial cells, in particular endothelial cell monolayer, wherein for example endothelial cells, in particular endothelial cell monolayer are injured by placing stents or other devices / implants into a vessel.

Moreover, the present invention refers to a pharmaceutical composition comprising an extract of Arthrospira, in particular spirulina for use in the prevention or treatment of a blood vessel disease selected from the group of neo-intimal hyperplasia, restenosis, thrombosis or embolism, wherein a medical device for implantation into a bodily vessel or luminal structure is coated with an extract of Arthrospira, in particular spirulina.

According to the present invention such a said blood vessel injury may result in further complications like bacterial infections, bloodstream infections, primary bacteremia and sepsis. As outlined in the examples and figures an extract of Arthrospira, in particular spirulina is effective against bacterial toxins like lipopolysaccharides.

In particular, the present invention refers to a pharmaceutical composition comprising an extract of Arthrospira, in particular spirulina for use in the prevention or treatment of bacterial infections, preferably bloodstream infections, primary bacteremia and sepsis.

Moreover, the present invention refers to a pharmaceutical composition comprising an extract of Arthrospira, in particular spirulina for use in the prevention or treatment of a disease selected from the group of bacterial infections, preferably bloodstream infections, primary bacteremia and sepsis, wherein a medical device for implantation into a bodily vessel or luminal structure is coated with an extract of Arthrospira, in particular spirulina.

All mentioned diseases and defects are well described in Pschyrembel (e.g. https://www.pschyrembel.de/).

The active agents, i.e. an extract of Arthrospira, in particular spirulina may be administered by conventional methods for solid drug preparations mixing e.g. all active agents and pelletizing them for example into pellets together with conventional excipients or auxiliary materials.

The pharmaceutical preparations may be administered in liquid or solid form for oral, enteral or parenteral application including intravenous routes. In this connection all conventional forms of application are possible, in particular it is available in a galenic formulation like tablets, coated tablets, pellets, capsules, dragees, sirups, solutions, suspensions. Preferably, water is used as an injection medium containing added substances common in injection solutions such as stabilizers, dissolving intermediaries and buffers. If desired, preparations suited for oral application may contain flavorings or sweeteners.

As used herein the terms "treating" and "treatment" or "preventing" and "prevention" refer to any and all uses which remedy a condition or symptoms, prevent the establishment of a condition or disease, or otherwise prevent, hinder, retard, or reverse the progression of a condition or disease or other undesirable symptoms in any way whatsoever. Thus, the terms "treating" and "treatment" or "preventing" and "prevention" are to be considered in their broadest context. For example, treatment does not necessarily imply that a subject is treated until total recovery.

As used herein the term "subject" includes humans and animals. Typically, the subject is a human, or a patient.

In the following, the present invention is described in more detail by way of examples and figures. However, these examples are not intended to limit the scope of protection of the present invention in any way.

EXAMPLES

**Example 1: Protocol for Cell Index (CI) measurement by RT-CES system**

The xCELLigence system is available from ACEA Biosciences, San Diego, CA, USA: It consists of a plate station with up to six 96 well E-Plates and a software for automatic and real-time data acquisition and display. The system measures electrical impedance across micro-electrodes integrated on the bottom of tissue culture E-Plates. It allows to monitor changes of adherence, spreading and proliferation of HUVEC (human umbilical vascular endothelial cells) or other cells in real time based on the measured cell-electrode impedance. From these data, a parameter termed "Cell Index (CI)" can be calculated, according to

$$CI = \max_{i = 1,..,N} \left[ \left( \frac{Rcell(fi)}{Rb(fi)} - 1 \right) \right]$$

where $R_b(f)$ and $R_{cell}(f)$ are the frequency dependent electrode resistances (a component of the impedance) without cells or with cells present, respectively. N is the number of the frequency points at which the impedance is measured.

Thus, CI is a quantitative measure of the status of the cells in an electrode-containing well. Under the same physiological conditions, more cells attached on to the electrodes leads to larger $R_{cell}(f)$ value, leading to a larger value for CI. Furthermore, for the same number of cells present in the well, a change in the morphology of the cells (spread cells) will also lead to a change in the CI. A "Normalized Cell Index" at a given time point is calculated by dividing the Cell Index at the time point by the Cell Index at a reference time point. Thus, the normalized Cell Index is 1 at the reference time point.

In the absence of living cells (cell culture medium only) or with a suspension of dead cells, the cell index values will be close to zero. After cellular attachment onto the electrode, the measured signal correlates linearly with cell number throughout the experiment with sufficient accuracy, which has been shown in many publications [see e.g. 11].

**Example 2: Cultivation of HUVEC and preparation of SP extract**

*Endothelial Cells*

Human vein endothelial cells (HUVEC) used in this study were purchased from Lonza (Basel, Switzerland). HUVEC were cultured in a standard humidified incubator at 37 °C with 5% $CO_2$ according to optimal media and growth conditions. Cells were used at passage 4 for experiments.

*Preparation Spirulina extract (in short: SP) for cell culture experiments*

Spirulina powder (Biospirulina, Sanatur GmbH, Singen, Germany) was stirred overnight in sterile 0.9% NaCl solution (B.Braun, Melsungen, Germany) at room temperature (10 mg / ml). Then the extract was centrifuged at 3400g for 5 minutes with subsequent filtration using a 0.45 and 0.22 $\mu$m filter. The extract was stored at 4 °C until further processing.

**Example 3: Influence of SP on HUVEC monolayer formation and its influence on lipopolysaccharides (in short: LPS)-mediated cytotoxicity**

*Study design*

The experiments were designed as prospective, controlled *in vitro* study using human umbilical vein endothelial cells (HUVEC). Optimal HUVEC number was determined in a set of experiments in order to obtain a significant cell index value and a constant cell growth during the entire duration of the experiment. From these experiments the optimum cell density was chosen as 3000 cells/well.

*Influence of SP on HUVEC monolayer formation*

Thereafter, the intrinsic effect of SP in increasing concentrations (0.125 $\mu$g/ml, 50 $\mu$g/ml, 250 $\mu$g/ml) on viability, adhesion and proliferation of human vein endothelial cells was assessed in real-time by the xCELLigence system E-plate 16 (ACEA Biosciences, Inc.) for 80 h at 10 min intervals.

Depending on the concentration of SP an increase of the cell index was observed 56 h after adding Spirulina extract (SP). The cell index (CI) after the addition of 0.125 $\mu$g/ml Spirulina was CI=4.53, after adding 50 $\mu$g/ml CI=5.25 and CI=4.97 for the addition of 250 $\mu$g/ml SP.

Table 1: Tukey-Kramer significance levels for Spirulina platensis supplementation.

|                  | Control | 0.125 $\mu$g/ml | 50 $\mu$g/ml | 250 $\mu$g/ml |
|------------------|---------|-----------------|--------------|---------------|
| Control          | -       | -               | 0.05         | 0.05          |
| 0.125 $\mu$g/ml  |         | -               | 0.05         | -             |
| 50 $\mu$g/ml     |         |                 | -            | -             |
| 250 $\mu$g/ml    |         |                 |              | -             |

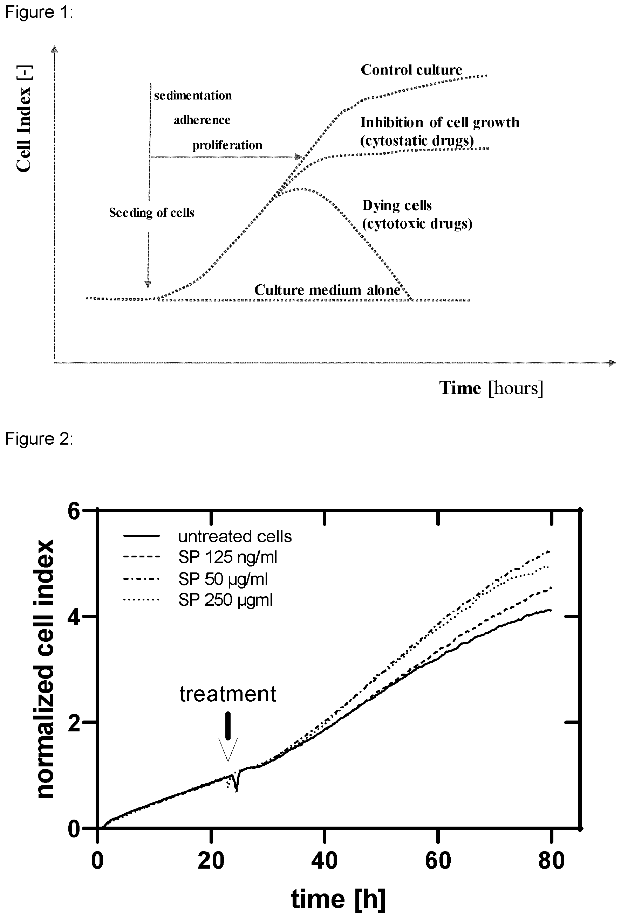

Figure 2 shows the HUVEC density after supplementation of the cell culture medium with different concentrations of SP in comparison to untreated HUVEC over cultivation time.

*Influence of lipopolysaccharides (in short: LPS) on HUVEC monolayer formation*

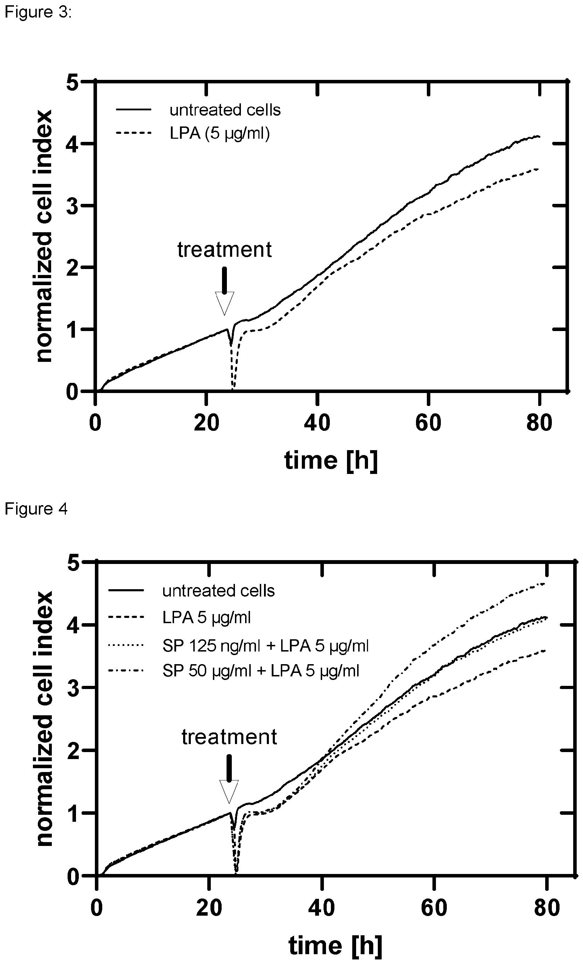

By adding LPS of E. coli (5 $\mu$g/ml, Merck KGaA, Darmstadt, Germany [product L6638, dissolved in DMSO], hereinafter: LPA) as toxic component of bacterial lipopolysaccharides (LPS), a significant and concentration-dependent decrease of the HUVEC monolayer density was observed. The CI was diminished after the addition of 5 $\mu$g/ml LPA to the cell culture medium to 3.61 compared to 4.155 of untreated HUVEC. It became obvious that most of the HUVEC detached immediately after LPA treatment for a short time period (see CI figure 3 below the arrow "treatment"), followed by renewed attachment. This effect was not seen for untreated HUVEC or HUVEC treated with SP (Figure 1 and 2) although the same volumes (medium or medium with SP extract) were added to the cells. This seemed to be a specific effect of LPA.

The two samples differed significantly (ANOVA: $p < 0.001$). Tukey-Kramer test showed that the control culture differed from the HUVEC supplemented with 5 μg/ml LPA (p<0.05).

Table 2: Tukey-Kramer significance levels for LPA supplementation in [μg/ml].

|  | Control | 5 μg/ml LPA |
| --- | --- | --- |
| Control | - | 0.05 |

Figure 3 shows the HUVEC density after supplementation of the cell culture medium with 5μg/ml LPA in comparison to untreated HUVEC over cultivation time.

*Effect of SP on endothelial cell impairment by LPA (5 μg/ml)*

The addition of 5 μg/ml LPA to the cell culture medium (red curve) induced - as already seen in figure 3 - a substantial decrease of adherent HUVEC compared to control cells from 4.155 to 3.61 56 hours after adding LPA. This decline was completely compensated by the initial addition of 0.125 μg/ml SP. A harmful effect of LPA on HUVEC was not observed anymore.

After the supplementation of the cell culture medium with 50 μg/ml SP and then adding 5 μg/ml LPA even more HUVEC adhered/proliferated compared to untreated control cells (Figure 4). So in this case, a suitable concentration of SP even overcompensated the toxic effect of LPA.

Table 3: Tukey-Kramer significance levels for LPA supplementation.

|  | Control | 5 μg/ml LPA | 5 μg/ml LPA+0.125μg/ml SP | 5 μg/ml LPA+50μg/ml SP |
| --- | --- | --- | --- | --- |
| Control | - | 0.05 | - | - |
| 5 μg/ml LPA |  | - | - | 0.05 |
| 5 μg/ml LPA+0.125μg SP |  |  | - | 0.05 |
| 5 μg/ml LPA+50μg SP |  |  |  | - |

Figure 4 shows the development of HUVEC densities over time after supplementation of the cell culture medium with 5 μg/ml LPA in absence or presence of different SP concentrations.

The study revealed that the aqueous extract of Spirulina i.) led - in comparison to control cells - to an accelerated formation of an endothelial cell monolayer and ii.) had a protective effect against endothelial cell detachment due to LPS and depending on the SP-concentration of the extract. The harmful effect of LPA could be reversed by a concentration of 0.125 μg/ml spirulina, and at a concentration of 50 μg/ml there was an accelerated endothelialization despite LPA.

FIGURES:

Figure 1: Sketch of measurements of the Cellular Index under different conditions over time.

Figure 2: Course of mean normalized Cell Index curves during the cultivation of control HUVEC), after adding 0.125 μg/ml SP, 50 μg/ml SP, 250 μg/ml SP or 1000 μg/ml SP (symbols for each defined in graph). Presented are graphs of arithmetic means for n=7 experiments (each treatment).

Figure 3: Mean normalized Cell Index curves of untreated HUVEC monolayer formation in cell culture wells compared to LPA-treated HUVEC during the cultivation time of 80 hours. Presented are graphs of arithmetic means of n=7 experiments per treatment

Figure 4: Effect of SP on LPA-induced (5 μg/ml) HUVEC impairment/-detachment compared to control cells during the cultivation time of 80 hours. Mean normalized CI of control cultures, CI of HUVEC treated with 5 μg/ml LPA, CI of HUVEC treated with 0.125 μg/ml SP + 5 μg/ml LPA, CI of HUVEC treated with 50 μg/ml SP + 5 μg/ml (curve symbols defined in graph). Presented are graphs of arithmetic means of n=7 experiments per treatment

References

[1] Jung F, Krüger-Genge A, Waldeck P, J-H. Küpper J-H. Spirulina platensis, a super food? J Cell Biotechnol. 2019;5:43-54.

[2] Khan Z, Bhadouria P, Bisen PS. Nutritional and therapeutic potential of Spirulina. Curr Pharm Biotechnol. 2005;6:373-379.

[3] Miczke A, Szulinska M, Hansdorfer-Korzon R, Kregielska-Narozna M, Suliburska J, Walkowiak J, Bogdanski P. Effects of spirulina consumption on body weight, blood pressure, and endothelial function in overweight hypertensive Caucasians: a double-blind, placebo-controlled, randomized trial. European Review for Medical and Pharmacological Sciences. 2016;20: 150-6.

[4] Kuhad A, Tirkey N, Pilkhwal S, Chopra K. 2006a. Renoprotective effect of Spirulina fusiform is on cisplatin-induced oxidative stress and renal dysfunction in rats. Ren Fail.2006 28: 247-254. 2016;20:150-156.

[5] Dartsch PC. Antioxidant Potential of Selected Spirulina platensis Preparations. Phytother Res. 2008;22,627-633.

[6] Chu W-L, Lim Y-W, Radhakrishnan AK, Lim PE. Protective effect of aqueous extract from Spirulina platensis against cell death induced by free radicals. BMC Complement Altern Med. 2010;10:53.

[7] Bhat VB, Madyastha KM. Scavenging of peroxynitrite by phycocyanin and phycocyanobilin from Spirulina platensis: protection against oxidative damage to DNA. Biochem Biophys Res Commun. 2001; 285:262-266.

[8] Pinero Estrada JE, Bermejo Bescos P, Villar del Fresno AM. Antioxidant activity of different fractions of Spirulina platensis protean extract. Farmaco. 2001;56:497-500.

[9] Heo S-Y, Ko SC, Kim CS, Oh G-W, Ryu B, Qian Z-J, Kim G, Park WS, Choi W-I, Phan TTV, Heo S-J, Kang D-H, Yi M, Jung W-K. A heptameric peptide purified from Spirulina sp. gastrointestinal hydrolysate inhibits angiotensin I-converting enzyme- and angiotensin II-induced vascular dysfunction in human endothelial cells. International Journal of Molecular Medicine. 2017;39:1072-1082.

[10] Viswanadha VP, Sivan S, Rajendra Shenoi R. (2011). Protective effect of Spirulina against 4-nitroquinoline-1-oxide induced toxicity. Mol Biol Rep 38:309-17.

[11] Kho D, MacDonald C, Johnson R, Unsworth CP, Carroll SJ, Mez ED, Angel CE, Graham ES. Application of xCELLigence RTCA Biosensor Technology for Revealing the Profile and Window of Drug Responsiveness in Real Time. 2015, 5, 199-222.

**Claims**

1. Pharmaceutical composition comprising an extract of Arthrospira or spirulina for use in the prevention or treatment of a blood vessel disease selected from the group of neo-intimal hyperplasia, restenosis, thrombosis or embolism.

2. Pharmaceutical composition comprising an extract of Arthrospira or spirulina for use in the prevention or treatment of a disease selected from the group of bacterial infections, bloodstream infections, primary bacteremia and sepsis.

3. Pharmaceutical composition comprising an extract of Arthrospira or spirulina for use in the regeneration or re-endothelization and / or acceleration of forming endothelial cells or endothelial cell monolayer.

4. Pharmaceutical composition comprising an extract of Arthrospira or spirulina for use in the prevention or treatment of a disease according to claim 1 or claim 2 or use according to claim 3, wherein a medical device for implantation into a bodily vessel or luminal structure is coated with an extract of Arthrospira or spirulina.

5. A medical device for implantation into a bodily vessel or luminal structure, wherein said medical device has a coating and the coating comprises an extract of Arthrospira or spirulina.

6. A medical device for implantation into a bodily vessel or luminal structure according to claim 5, wherein the implantable device is selected from the group of stents, stent-grafts, synthetic bypass grafts, embolic filters, occluder systems, detachable coils, pacemaker and defibrillator leads, plates, screws, spinal cages, dental implants, ventricular assist devices, artificial hearts, artificial heart valves, annuloplasty devices, artificial joints, and implantable sensors.

7. Pharmaceutical composition in accordance with one of the claims 1 to 4, **characterized in that** it is available in a galenic formulation, especially an oral formulation, such as tablets, coated tablets, pellets, capsules, dragees, sirups, solutions, suspensions.

8. Pharmaceutical composition or a medical device in accordance with one of the preceding claims, wherein Arthrospira is selected from the species of A. platensis, A. maxima and A. fusiformis.

9. Pharmaceutical composition or a medical device in accordance with one of the preceding claims, wherein an extract of Arthrospira or spirulina is enriched with bioactive compounds by means of concentration, fractionation, or addition.

10. Pharmaceutical composition or a medical device in accordance with one of the preceding claims, wherein an extract of Arthrospira or spirulina is enriched with compounds selected from the group of secondary plant substances, photosynthetically active pigments, phycocyanin, xanthophyll, chlorophyll, beta-carotene, echinenone, xanthine, fatty acids, linolenic acid (ALA), linoleic acid, stearidonic acid (SDA), eicosapentaenoic acid (EPA), docosahexaenoic acid (DHA), arachidonic acid (AA), oligosaccharides, polyphenols.

Figure 1:

Figure 2:

Figure 3:

Figure 4

Europäisches Patentamt
European Patent Office
Office européen des brevets

# EUROPEAN SEARCH REPORT

Application Number

EP 20 19 2425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PARK A REUM ET AL: "Tri-layered silk fibroin and poly-[epsilon]-caprolactone small diameter vascular grafts testedin vitroandin vivo", MACROMOLECULAR RESEARCH, POLYMER SOCIETY OF KOREA, SEOUL, KR, vol. 23, no. 10, 29 October 2015 (2015-10-29), pages 924-936, XP035577058, ISSN: 1598-5032, DOI: 10.1007/S13233-015-3126-X [retrieved on 2015-10-29] | 1,3-5, 8-10 | INV. A61K35/748 A61P9/00 A61P31/04 A61L27/36 |
| Y | * abstract * | 1-10 | |
| X | US 2006/024328 A1 (PASCO DAVID [US] ET AL) 2 February 2006 (2006-02-02) * claims 1, 2, 9,30; examples 1, 4, 9 * | 2,7-10 | |
| X | GB 2 568 554 A (FOURTEEN FIVE LTD [GB]; RUBINA MUGHAL [GB]) 22 May 2019 (2019-05-22) * claims * | 2,4,5, 7-10 | |
| Y | MAGDA A. FURMANIAK ET AL: "Edible Cyanobacterial Genus Arthrospira: Actual State of the Art in Cultivation Methods, Genetics, and Application in Medicine", FRONTIERS IN MICROBIOLOGY, vol. 8, 18 December 2017 (2017-12-18), XP055771468, Lausanne ISSN: 1664-302X, DOI: 10.3389/fmicb.2017.02541 * page 12, left-hand column, last paragraph - right-hand column, paragraph 3; table 2 * * page 13, right-hand column, paragraph 7 - page 14, left-hand column, paragraph 1 * | 1-10 | TECHNICAL FIELDS SEARCHED (IPC) A61K A61P A61L |

The present search report has been drawn up for all claims

| Place of search | Date of completion of the search | Examiner |
|---|---|---|
| Munich | 3 February 2021 | Winger, Rudolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 960 193 A1**

## ANNEX TO THE EUROPEAN SEARCH REPORT
## ON EUROPEAN PATENT APPLICATION NO.

EP 20 19 2425

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2021

| Patent document cited in search report | | | Publication date | Patent family member(s) | | | Publication date |
|---|---|---|---|---|---|---|---|
| US 2006024328 | A1 | | 02-02-2006 | EP | 1778260 | A2 | 02-05-2007 |
| | | | | US | 2006024328 | A1 | 02-02-2006 |
| | | | | WO | 2006015115 | A2 | 09-02-2006 |
| GB 2568554 | A | | 22-05-2019 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JUNG F ; KRÜGER-GENGE A ; WALDECK P ; J-H. KÜPPER J-H.** Spirulina platensis, a super food?. *J Cell Biotechnol,* 2019, vol. 5, 43-54
- **KHAN Z ; BHADOURIA P ; BISEN PS.** Nutritional and therapeutic potential of Spirulina. *Curr Pharm Biotechnol,* 2005, vol. 6, 373-379
- **MICZKE A ; SZULINSKA M ; HANSDORFER-KORZON R ; KREGIELSKA-NAROZNA M ; SULIBURSKA J ; WALKOWIAK J ; BOGDANSKI P.** Effects of spirulina consumption on body weight, blood pressure, and endothelial function in overweight hypertensive Caucasians: a double-blind, placebo-controlled, randomized trial. *European Review for Medical and Pharmacological Sciences,* 2016, vol. 20, 150-6
- **KUHAD A ; TIRKEY N ; PILKHWAL S ; CHOPRA K.** 2006a. Renoprotective effect of Spirulina fusiform is on cisplatin-induced oxidative stress and renal dysfunction in rats. *Ren Fail,* 2006, vol. 28, 247-254
- *REN FAIL,* 2016, vol. 20, 150-156
- **DARTSCH PC.** Antioxidant Potential of Selected Spirulina platensis Preparations. *Phytother Res.,* 2008, vol. 22, 627-633
- **CHU W-L ; LIM Y-W ; RADHAKRISHNAN AK ; LIM PE.** Protective effect of aqueous extract from Spirulina platensis against cell death induced by free radicals. *BMC Complement Altern Med,* 2010, vol. 10, 53

- **BHAT VB ; MADYASTHA KM.** Scavenging of peroxynitrite by phycocyanin and phycocyanobilin from Spirulina platensis: protection against oxidative damage to DNA. *Biochem Biophys Res Commun,* 2001, vol. 285, 262-266
- **PINERO ESTRADA JE ; BERMEJO BESCOS P ; VILLAR DEL FRESNO AM.** Antioxidant activity of different fractions of Spirulina platensis protean extract. *Farmaco,* 2001, vol. 56, 497-500
- **HEO S-Y ; KO SC ; KIM CS ; OH G-W ; RYU B ; QIAN Z-J ; KIM G ; PARK WS ; CHOI W-I ; PHAN TTV.** A heptameric peptide purified from Spirulina sp. gastrointestinal hydrolysate inhibits angiotensin I-converting enzyme- and angiotensin II-induced vascular dysfunction in human endothelial cells. *International Journal of Molecular Medicine,* 2017, vol. 39, 1072-1082
- **VISWANADHA VP ; SIVAN S ; RAJENDRA SHENOI R.** Protective effect of Spirulina against 4-nitroquinoline-1-oxide induced toxicity. *Mol Biol Rep,* 2011, vol. 38, 309-17
- **KHO D ; MACDONALD C ; JOHNSON R ; UNSWORTH CP ; CARROLL SJ ; MEZ ED ; ANGEL CE ; GRAHAM ES.** Application of xCELLigence RTCA Biosensor Technology for Revealing the Profile and Window of Drug Responsiveness. *Real Time,* 2015, vol. 5, 199-222